# EUROPEAN PATENT APPLICATION

(11) **EP 2 022 445 A1**
(43) Date of publication of application: **11.02.2009**
(21) Application number: 07740890.4
(22) Date of filing: 03.04.2007
(51) Int. Cl.: A61D 7/04, A01K 67/00, A61M 16/01

(54) **ANESTHETIC SYSTEM FOR SMALL ANIMAL**

(30) Priority: 10.05.2006 JP 2006131744
(71) Applicant: S.K.I.Net Inc., Bunkyo-ku, Toyko 113-0034 (JP)
(72) Inventor: INOUE, Masaaki, Bunkyo-ku, Tokyo 113-0034 (JP); ASAKURA, Takekazu, Bunkyo-ku, Tokyo 113-0034 (JP); KAKINUMA, Akio, Bunkyo-ku, Tokyo 113-0033 (JP); NATSUME, Katsuhiko, Bunkyo-ku, Tokyo 113-8551 (JP); HIRABAYASHI, Shirokazu, Bunkyo-ku, Tokyo 113-0034 (JP)
(74) Representative: Hallybone, Huw George
(86) International application number: PCT/JP2007/057454
(87) International publication number: WO 2007/129515

(57) **Abstract**

An anesthesia system includes a main unit (10) for supplying anesthetic gas and providing artificial respiration, an induction chamber (20) for receiving a small animal and for introducing the anesthetic gas, an anesthetic gas treatment section (30) connected to the induction chamber (20) and a breathing circuit tube (50) having an intubation portion (53). The main unit (10) has a first outlet port (10a), a second outlet port (10c), a return port (10b) and a communication passage (17) connecting the return port and the second outlet port. The anesthetic gas exiting through the first outlet port (10a) is conveyed through the breathing circuit tube (50) to return to the return port (10b) and further conveyed through the communication passage (17), the second outlet port (10c) and a supply tube (60) into the induction chamber (20).

## Description

### TECHNICAL FIELD

This invention relates to an anesthesia system for administering anesthetic to a small animal such as a rat and a mouse while at the same time providing artificial respiration to the subject small animal.

### BACKGROUND ART

It is well known in the art to perform a variety of experiments or operations on a small animal while administering anesthetic to and providing artificial respiration to the subject small animal. One example of an anesthesia system for such a purpose is disclosed in FIG. 3 of the patent document 1 (Japanese Unexamined Patent Application Publication No. 2005-279218), which is filed by the same applicant. The anesthesia system is described hereinafter using reference numerals used in the patent document 1. The anesthesia system includes an anesthetic gas supply section including, from upstream to downstream, an air pump 1, a mass flow controller 2 and a vaporizer 3 for vaporizing liquid anesthetic.

The anesthesia system further includes a breathing circuit 4. An intubation portion 9 to be inserted in a trachea of a small animal is connected to some point in the breathing circuit 4. Anesthetic gas from the anesthetic gas supply section is supplied to the small animal through an inhalation side of the breathing circuit 4 and the intubation portion 9. A pressure sensor 6 and an exhalation valve 5 are connected to an exhalation side of the breathing circuit 4. When the exhalation valve 5 is closed, the anesthetic gas is supplied to the small animal, and the small animal is made to inhale the gas. When the pressure sensor 6 detects a pre-set level of pressure, the exhalation valve 5 is opened, and the small animal is made to exhale. Closing of the exhalation valve 5 is performed periodically, whereby artificial respiration is provided to the small animal while anesthetic is administered to the small animal.

The anesthesia system further includes an induction chamber 11 for receiving the small animal. The induction chamber 11 is connected to a downstream end of a branch passage that branches from the inhalation side of the breathing circuit 4. A switching valve 10 is disposed at a point where the branch passage branches from the breathing circuit 4. When the branch passage is selected at the switching valve 10, the anesthetic gas is conveyed to the induction chamber 11, and anesthetic can be administered to the small animal received in the induction chamber 11.

The anesthesia system further includes absorption columns 12, 13 for absorbing anesthetic gas, respectively connected to an outlet of the exhalation valve 5 and the induction chamber 11. This arrangement avoids or reduces emission of the anesthetic gas from the exhalation valve 5 or the induction chamber 11 to the laboratory room.

### DISCLOSURE OF THE INVENTION

### PROBLEM TO BE SOLVED BY THE INVENTION

However, in the anesthesia system illustrated in FIG. 3 of the Patent Document 1 given above, treatment of waste anesthetic gas is required both at the outlet of the exhalation valve 5 and at the induction chamber 11. In this system, complicated structures are required for treatment of waste anesthetic gas.

### MEANS FOR SOLVING THE PROBLEM

In order to solve the above mentioned problem, the present invention provides an anesthesia system comprising:
(a) a main unit having a first outlet port, a second outlet port, a return port and a communication passage connecting the return port and the second outlet port, the main unit supplying anesthetic gas through the first outlet port and providing artificial respiration;
(b) an induction chamber for induction of the anesthetic gas, the induction chamber being able to receive a small animal therein;
(c) an anesthetic gas treatment section connected to the induction chamber;
(d) a breathing circuit tube having an inhalation portion, an exhalation portion and an intubation portion connected to a downstream end of the inhalation portion and an upstream end of the exhalation portion, the inhalation portion having an upstream end connected to the first outlet port of the main unit, the exhalation portion having a downstream end connected to the return port of the main unit; and
(e) a supply tube having an upstream end connected to the second outlet port of the main unit and having a downstream end connected to the induction chamber, the supply tube conveying the anesthetic gas from the second outlet port to the induction chamber.

In the above mentioned arrangement, the anesthetic gas is conveyed from the first outlet port of the main unit to the induction chamber through the breathing circuit tube, the return port, the communication passage, the second outlet port, and the supply tube, and can be treated at the anesthetic gas treatment section. In this arrangement, structure for treatment of waste anesthetic gas is simplified.

Preferably, the main unit comprises a first passage connected to the first outlet port and a second passage connected to the second outlet port, and the anesthetic gas is selectively supplied either through the first passage or through the second passage. In this arrangement, the anesthetic gas can be supplied to the induction chamber directly through the second outlet port and the supply tube without passing through the breathing circuit tube. This allows for effective administration of anesthetic to the small animal in the induction chamber before intubation, etc.

Preferably, the main unit has a main passage connected to the first outlet port, the main passage is provided with an anesthetic gas supply section for supplying the anesthetic gas, the communication passage is provided with an exhalation valve for opening and closing the communication passage, and the artificial respiration is performed by repeatedly opening and closing the exhalation valve.

Preferably, the main unit has a branch passage branched from the main passage at a branching point in a downstream side of the anesthetic gas supply section and connected to the second outlet port. Preferably, the main unit further has a switching valve provided at the branching point, and the switching valve selects between a downstream portion of the main passage connected to the first outlet port and the branch passage connected to the second outlet port. In this arrangement, the anesthetic gas can be supplied to the induction chamber directly through the branch passage, the second outlet port and the supply tube without passing through the breathing circuit tube. This allows for effective administration of anesthetic to the small animal in the induction chamber before intubation, etc.

Preferably, the anesthetic gas supply section has an air pump, a flow control section and a liquid anesthetic vaporizing section, wherein the air pump, the flow control section and the liquid anesthetic vaporizing section are disposed in the main passage and arranged from upstream to downstream of the main passage. Preferably, the anesthetic gas supply section further has a bypass passage connected to the main passage bypassing the flow control section and a bypass valve disposed in the bypass passage for opening and closing the bypass passage. In this arrangement, closing of the bypass valve allows for supply of the anesthetic gas at a flow rate controlled by the flow control section, which may be suitable for artificial respiration. On the other hand, opening of the bypass valve allows for supply of the anesthetic gas at a high flow rate, which may be suitable for administering anesthetic to the small animal in the induction chamber.

Preferably, a mask for covering a nose of a small animal is attached to a downstream end of the supply tube and the downstream end of the supply tube is removably connected to the induction chamber through the mask. In this arrangement, the anesthetic gas can be supplied to the induction chamber through the mask to anesthetize the small animal in the induction chamber. If necessary, the anesthetic gas can be delivered to the small animal through the mask removed from the induction chamber and placed over the nose of the small animal.

Preferably, the induction chamber has a chamber main body having a top opening and a closure for opening and closing the top opening. Preferably, the downstream end of the supply tube is connected to the induction chamber through the mask received in the induction chamber. In this arrangement, the mask can be easily placed into and removed from the induction chamber by opening the closure.

Preferably, the chamber main body has a notch formed in an upper edge of a peripheral wall thereof, the notch being provided for receiving the supply tube. In this arrangement, leakage of the anesthetic gas from the induction chamber can be avoided or reduced when the mask is placed in the induction chamber and the closure is closed.

Preferably, the closure of the induction chamber has a notch formed in an edge thereof, the notch being provided for receiving the supply tube. In this arrangement, leakage of the anesthetic gas from the induction chamber can be avoided or reduced when the mask is placed in the induction chamber and the closure is closed.

Preferably, the chamber main body has a divider plate removably disposed therein, and a small space is defined in the chamber main body with the divider plate. In this arrangement, with the divider plate removed, the induction chamber may be suitable for housing a rat, etc., and with the divider plate attached, the small space of the induction chamber may be suitable for receiving a mouse, etc.

Preferably, the anesthesia system further comprises an intubation table unit for placing a small animal thereon for insertion of an intubation tube to a trachea of the small animal. Preferably, one end of a bendable arm is attached to the intubation table unit, and a clamping tool for removably clamping the mask is disposed on the other end of the arm. In this arrangement, the mask covering the nose of the small animal is supported by the arm. This allows an operator to take his or her hand off the mask, thereby improving the workability.

### EFFECT OF THE INVENTION

According to the present invention, structure for treatment of waste anesthetic gas in an anesthesia system for a small animal can be simplified.

### BRIEF DESCRIPTION OF THE DRAWINGS

FIG. 1 is a schematic drawing of one embodiment of an anesthesia system according to the present invention.
FIG. 2 is a circuit diagram of an inner structure of a main unit of the anesthesia system.
FIG. 3 is a partially broken-away perspective view of an induction chamber of the anesthesia system.
FIG. 4 is an enlarged longitudinal sectional view of a part of the induction chamber.
FIG. 5 is a perspective view of an intubation table unit of the anesthesia system.
FIG. 6 is a plan view of the intubation table unit with arms attached thereto.
FIG. 7 is a plan view of the intubation table unit with first support blocks, a second support block and a hooking tool as well as the arms attached thereto.
FIG. 8 is a cross-sectional view of FIG. 7 taken along the line VIII-VIII.
FIG. 9 is a side view of the first support block.
FIG. 10 is a plan view of the intubation table unit with other first support blocks and another second support block attached thereto.
FIG. 11 is a side view of a laryngoscope used for intubation.
FIG. 12 is a rear view of the laryngoscope.
FIG. 13 is a schematic drawing illustrating a step of administering anesthesia to a small animal housed in the induction chamber.
FIG. 14 is a schematic drawing illustrating a step of administering anesthesia to the small animal placed on the intubation table unit through a mask covering a nose of the small animal.
FIG. 15 is a schematic drawing illustrating a step of intubation performed on the small animal on the intubation table unit.
FIG. 16 is a schematic drawing illustrating a step of placing the intubated small animal on a laboratory table.
FIG. 17 is a schematic drawing illustrating an example of a step of repeated anesthesia in a case where the small animal wakes up.
FIG. 18 is a schematic drawing illustrating another example of a step of repeated anesthesia in a case where the small animal wakes up.
FIG. 19 is a schematic drawing illustrating still another example of a step of repeated anesthesia in a case where the small animal wakes up.
FIG. 20 is a partially cut-away perspective view of another example of the induction chamber.
FIG. 21 is a perspective view of still another example of the induction chamber.

### DESCRIPTION OF THE REFERENCE NUMERALS

- 10: main unit
- 10a: first outlet port
- 10b: return port
- 10c: second outlet port
- 11: main passage
- 11a: downstream portion of the main passage
- 12b: air pump
- 12c: mass flow controller (flow control section)
- 13: bypass passage
- 14a: vaporizer (liquid anesthetic vaporizing section)
- 15: anesthetic gas supply section
- 16: branch passage
- 17: communication passage
- 20, 20A, 20B: induction chamber
- 21: chamber main body
- 21a: notch
- 22: closure
- 22x: notch
- 30: anesthetic gas absorber (anesthetic gas treatment section)
- 40: intubation table unit
- 42: support board
- 42a, 42b, 42c, 42d: longitudinal groove
- 45a: arm
- 45c: clamping tool
- 46: first support block
- 46a: protrusion
- 46b: support groove
- 47: second support block
- 50: breathing circuit tube
- 51: inhalation portion
- 52: exhalation portion
- 53: intubation portion
- 60: supply tube
- 61: mask
- SV1: bypass valve
- SV2: switching valve
- SV3: exhalation valve

### BEST MODE FOR CARRYING OUT THE INVENTION

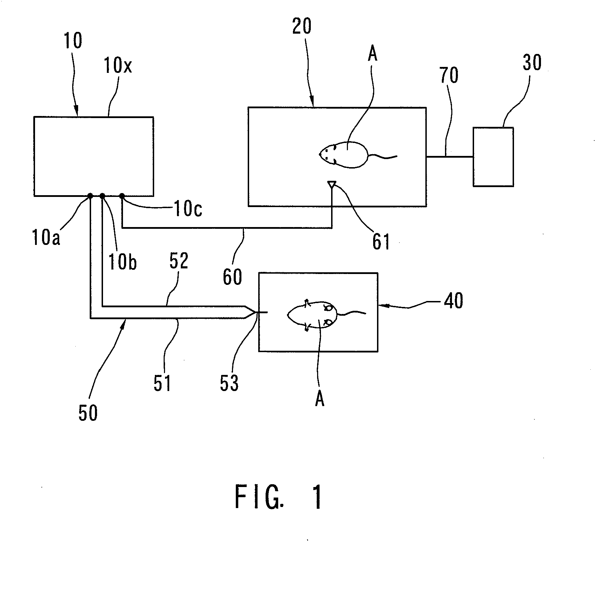

A first embodiment of the present invention will now be described with reference to FIGS. 1 to 19. Referring to FIG. 1, a small animal anesthesia system includes a main unit 10, an anesthesia induction chamber 20, an anesthetic gas absorber 30 (anesthetic gas treatment section), an intubation table unit 40, a breathing circuit tube 50, a supply tube 60 and an exhaust tube 70.

The main unit 10 has a housing 10x. Various components to be described later and piping structure connecting these components are disposed inside the housing 10x to constitute a unit. The housing 10x has a first outlet port 10a, a return port 10b and a second outlet port 10c. The housing 10x also has a power switch, an operational mode switch, an anesthesia switch, an artificial respiration switch, and a flow rate setting section (none is shown). A control section (not shown) for controlling the components inside the housing 10x in response to these switches is disposed inside the housing 10x.

The breathing circuit tube 50 has an inhalation portion 51 and an exhalation portion 52, both made of flexible resin tube, and a short intubation portion 53 connected to a downstream end of the inhalation portion 51 and an upstream end of the exhalation portion 52. An upstream end of the inhalation portion 51 is removably connected to the first outlet port 10a of the main unit 10 and a downstream end of the exhalation portion 52 is removably connected to the return port 10b. The intubation portion 53 is to be inserted into a trachea of a small animal as will be described later.

One end, i.e., upstream end, of the supply tube 60 is removably connected to the second outlet port 10c of the main unit 10 and a mask 61 is attached to the other end, i.e., downstream end, of the supply tube 60. The mask 61 can be removably housed in the induction chamber 20.
The exhaust tube 70 connects the induction chamber 20 and the anesthetic gas absorber 30. The anesthetic gas absorber 30 has anesthetic gas absorbent such as activated carbon therein.

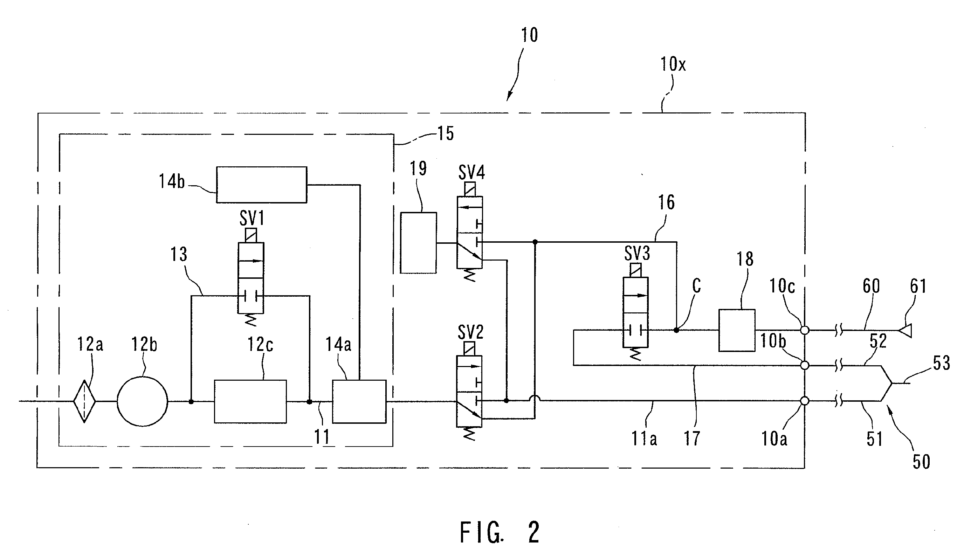

An inner structure of the main unit 10 will be described with reference to FIG. 2. Piping in the inner structure is referred to as passage in the description. The main unit 10 has a main passage 11. An air filter 12a, an air pump 12b and a mass flow controller 12c (flow control section) are disposed in the main passage 11 and are arranged from upstream to downstream of the main passage 11. A downstream end of the main passage 11 is connected to the first outlet port 10a.

A bypass passage 13 bypassing the mass flow controller 12c is connected to the main passage 11. A bypass valve SV1, which is a 2-way solenoid valve, is disposed in the bypass passage 13.

A vaporizer 14a (liquid anesthetic vaporizing section) is disposed in the main passage 11 at a location further downstream than the mass flow controller 12c. The liquid anesthetic is fed to the vaporizer 14a by an infusion pump 14b, which is a syringe pump, and vaporized by the vaporizer 14a. As a result, anesthetic gas, which is air containing vaporized anesthetic agents, flows out of the vaporizer 14a.

As will be understood from the above, the air filter 12a, the air pump 12b, the mass flow controller 12c, the bypass valve SV1, the vaporizer 14a and the infusion pump 14b constitute an anesthetic gas supply section 15.

An upstream end of a branch passage 16 is connected to the main passage 11 at a location in the downstream side of the vaporizer 14a. A downstream end of the branch passage 16 is connected to the second outlet port 10c. A switching valve SV2, which is a 3-way solenoid valve, is disposed at a connecting point of the main passage 11 and the branch passage 16. The gas from the anesthetic gas supply section 15 is sent either to the first outlet port 10a via a downstream portion 11a of the main passage 11 or to the second outlet port 10c via the branch passage 16, selectable by operation of the switching valve SV2.

The main unit 10 further includes a communication passage 17. One end, i.e., an upstream end, of the communication passage 17 is connected to the return port 10b. The other end, i.e., a downstream end, of the communication passage 17 is connected to the branch passage 16, and then to the second outlet port 10c. A connection point of the communication passage 17 and the branch passage 16 is denoted with "C" in the drawing. A portion of the branch passage 16 located further downstream than the connection point C also serves as a part of the communication passage 17.
A gas sensor 18 is disposed in the branch passage 16 at a location between the connection point C and the second outlet port 10c.

An exhalation valve SV3, which is a 2-way solenoid valve, is disposed in the communication passage 17 at a location between the return port 10b and the connection point C.
The main unit 10 further includes a pressure sensor 19. The pressure sensor 19 is selectively connected via an auxiliary switching valve SV4, which is a 3-way solenoid valve, either to the downstream portion 11a of the main passage 11 or to the branch passage 16.
The exhalation valve SV3 and the pressure sensor 19 serve as an artificial respirator portion of the main unit.

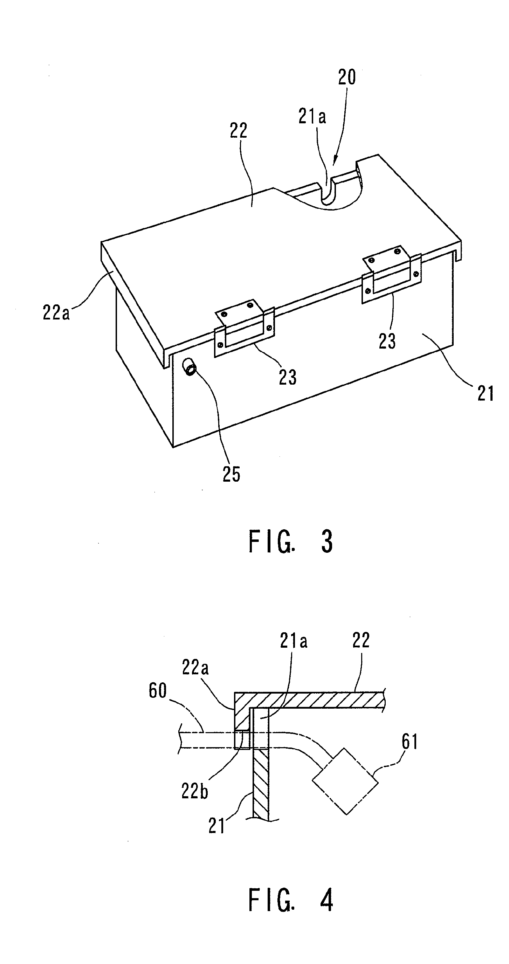

As shown in FIG. 3, the induction chamber 20 for housing a small animal has a rectangular parallelepiped configuration. The induction chamber 20 has a chamber main body 21 and a closure 22 for opening and closing a top opening of the chamber main body 21. The chamber main body 21 and the closure 22 are made of a transparent material. The closure 22 is connected to the chamber main body 21 via a hinge 23 at an edge of the closure 22. The closure 22 has flanges 22a formed in the other three edges thereof. The flanges 22a extend along side walls of the chamber main body 21.

As shown in FIGS. 3 and 4, the chamber main body 21 has a notch 21 a formed in a side wall thereof, to be more specific, in an upper edge of one of the three side walls to which the hinge 23 is not attached. A shallow notch 22b is formed in a lower edge of the flange 22a of the closure 22 at a location corresponding to the notch 21 a.

This arrangement allows the closure 22 to be closed, with the supply tube 60 inserted through the notch 21a of the chamber main body 21, and the mask 61 received in the chamber main body 21.
When the flanges 22a are not formed in the closure 22, only the notch 21a is formed in the chamber main body 21.

As shown in FIG. 3, a connecting terminal 25 is disposed in an upper portion of the side wall of the chamber main body 21. The connecting terminal 25 is to be connected with an upstream end of the exhaust tube 70.

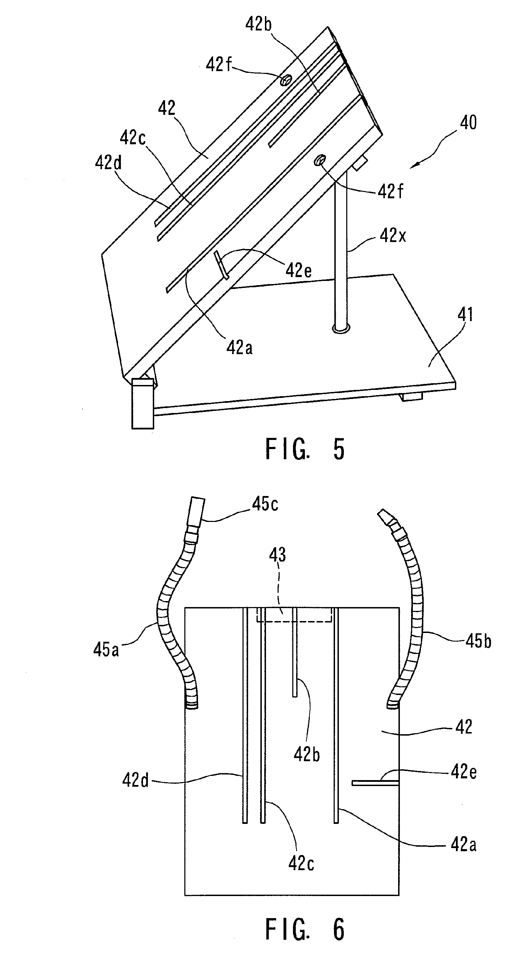

As shown in FIG. 5, the intubation table unit 40 has a horizontally extending base board 41, a support board 42 turnably connected to an edge of the base board 41 and a support pole 42x. An upper end of the support pole 42x is turnably connected to an undersurface of the support board 42. The support pole 42x is turned to assume a standing posture and a lower end of the support pole 42x is placed on the base board 41, thereby supporting the support board 42 with an inclination of at least 40 degrees, preferably 40 to 50 degrees, with respect to the base board 41.

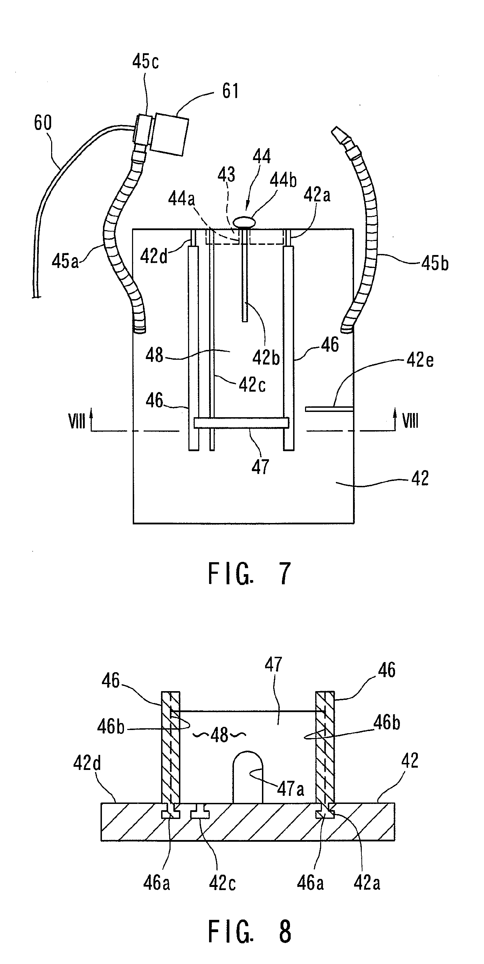

A plurality of, four, for example, longitudinal grooves 42a-42d extending linearly in the direction of inclination of the support board 42 and a transverse groove 42e extending in a direction orthogonal to the longitudinal grooves 42a-42d are formed in an upper surface of the support board 42. The grooves 42a-42e have a reverse T-shape cross sectional configuration as shown in FIG. 8. The longitudinal grooves 42a-42d reach an upper edge of the support board 42 and the transverse groove 42e reach a side edge of the support board 42. One of the longitudinal grooves 42b is shorter than the other longitudinal grooves.
A screw hole 42f is formed to the left and right of the longitudinal grooves 42a-42d in the upper surface of the support board 42.

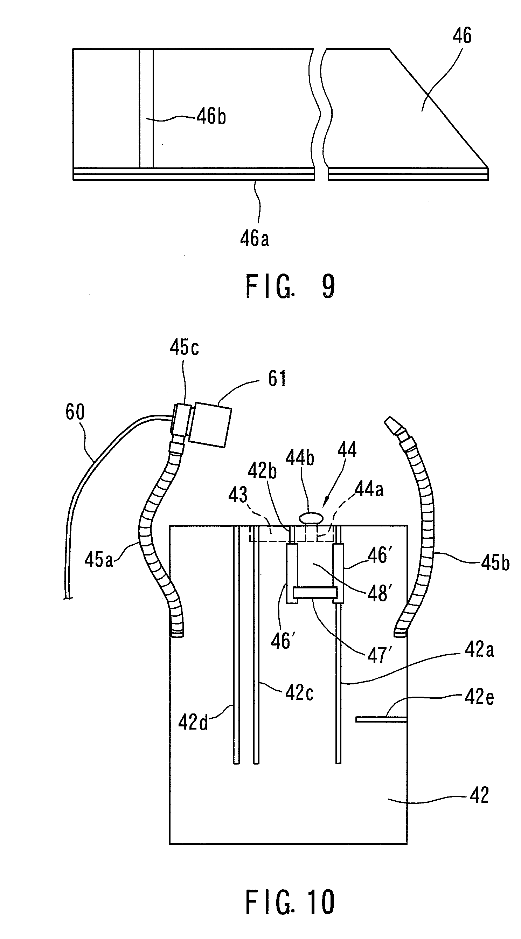

As shown in FIG. 6, a tape 43 extending along an upper edge of the support board 42 is attached to an undersurface of the upper edge of the support board 42.
As shown in FIGS. 7 and 10, a hooking tool 44 is detachably attached, i.e., positionally adjustably attached to the tape 43. The hooking tool 44 has a tape 44a and a rubber-made ring 44b attached to the tape 44a. The tapes 43, 44a cooperate to constitute a hook and loop faster. The ring 44b is provided for hooking upper front teeth of the small animal.

As shown in FIG. 6, lower ends of support arms 45a, 45b are removably screwed into the left and right screw holes 42f of the support board 42. The support arms 45a, 45b are bendable and have good shape maintainability. A clamping tool 45c having a structure similar to an ordinary clothespin is attached to an upper end of one of the arms 45a. As shown in FIGS. 7 and 10, the clamping tool 45c releasably clamps the mask 61. The other of the support arms 45b is provided as a spare. A lamp or another clamp for clamping a mask of a different size, for example, may be attached to an upper end of the other of the support arms 45b.

As shown in FIGS. 7 to 9, a pair of left and right first support blocks 46 and a second support block 47 may be removably attached to the support board 42 for supporting the small animal such as a rat.
The left and right first support blocks 46 have a configuration of an elongated plate. Each of the first support blocks 46 has a protrusion 46a having a reverse T-shape cross sectional configuration and extending along a lower edge of the first support block 46. A support groove 46b extending in a direction orthogonal to the protrusion 46a is formed in a side surface of the first support block 46 at a location in the vicinity of one end of the first support block 46.

The left and right first support blocks 46 are supported by the support board 42 in a standing posture with the protrusions 46a inserted in the longitudinal grooves 42a, 42d of the support board 42. The second support block 47 is inserted into the support grooves 46b of the left and right first support blocks 46 and supported in a standing posture. As a result, a space 48 for receiving the rat is formed by the support blocks 46, 47.
The second support block 47 is provided for supporting a weight of the rat. A notch 47a is formed in a lower edge of the second support block 47 so that a tail of the rat can be passed through the notch 47a.

FIGS. 7 and 8 show an example of the support board 42 with the support blocks 46, 47 attached in a way suitable for supporting the rat of a relatively big size. To support the rat of a relatively small size, the left and right first support blocks 46 are attached to the longitudinal grooves 42a, 42c of the support board 42. In this case, a second support block 47 having a smaller size than the one depicted in FIGS. 7 and 8 shall be used.

To support a mouse instead of a rat, other support blocks 46', 47' are used as shown in FIG. 10. The support blocks 46', 47' are smaller in size than the support blocks 46, 47, but are similar in shape to the support blocks 46, 47. Protrusions of left and right first support blocks 46' are inserted in the longitudinal grooves 42a, 42b of the support board 42. The support blocks 46', 47' form a smaller receiving space 48' for the mouse.
When the mouse is to be supported, a location of the hooking tool 44 is shifted to right.

A support block (not shown) for supporting tweezers or scissors, etc. may be attached to the transverse groove 42e of the support board 42 in a similar manner to the support blocks 46, 47.

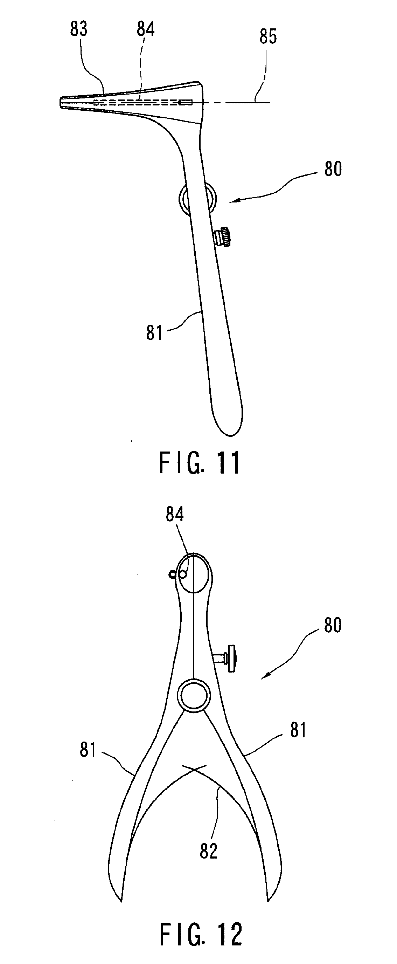

FIGS. 11 and 12 illustrate a laryngoscope 80 to be used for intubation of the small animal. The laryngoscope 80 has a pair of handles 81 rotatably connected to each other at a central portion thereof. A spring 82 is disposed between basal ends of the handles 81. The spring 82 biases distal ends of the handles 81 to closing directions. Each of the handles 81 has an insertion portion 83 formed in the distal end thereof. The insertion portion 83 has a shape of a beak extending in a direction generally orthogonal to the handle 81.

One of the insertion portions 83 has a tubal portion 84 formed in an inner surface thereof. The tubal portion 84 extends in a longitudinal direction of the insertion portion 83. A distal end of an optical fiber 85 is inserted in the tubal portion 84. A basal end of the optical fiber 85 is connected to a light source. The pair of the insertion portions 83 of the laryngoscope 80 is inserted in an oral cavity of the small animal and the insertion portions 83 are opened. An intubation tube, not shown, is inserted into a trachea of the small animal with an inside of the oral cavity of the small animal illuminated with light from the optical fiber 85.

Operation of the anesthesia system for administering anesthesia to and giving artificial respiration to the small animal will be described below.
Upon power-on, operation of each of the components of the main unit 10 is checked. After the checking of operation, four solenoid valves SV1, SV2, SV3, SV4 are returned to a non-energized condition as shown in FIG. 2. In this condition, the pressure sensor 19 communicates with atmosphere through the downstream portion 11a of the main passage 11, the inhalation portion 51 of the breathing circuit tube 50 and the intubation portion 53. Zero-point adjustment of the pressure sensor 19 is performed in this condition.

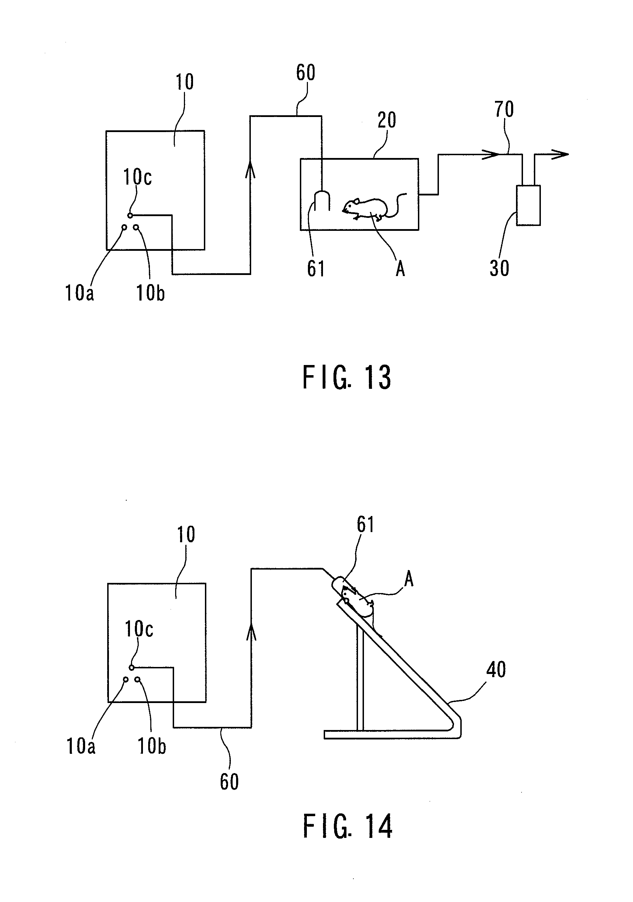

The small animal A is housed in the chamber main body 21 of the induction chamber 20 as shown in FIG. 13. The supply tube 60 is inserted through the notch 21a of the chamber main body 21, the mask 61 is received in the chamber main body 21 and the closure 22 is closed. In this way, the small animal A can be housed in the induction chamber 20 which is in a generally airtight condition.

The operational mode switch is set to an induction mode and the anesthesia switch is turned on. This activates the air pump 12b and causes the infusion pump 14b to feed the liquid anesthetic to the vaporizer 14a. As a result, the anesthetic gas is passed through the branch passage 16 via the switching valve SV2 in its off state. The anesthetic gas is further passed through the second outlet port 10c, the supply tube 60 and the mask 61 to be supplied into the induction chamber 20.

In the induction mode, the bypass valve SV1 is opened in its on state, thereby great amount of air is provided, and great amount of the liquid anesthetic is fed by the infusion pump 14b. This causes the induction chamber 20a to be filled with great amount of high-concentration anesthetic gas, thereby allowing the small animal A to be anesthetized in a short period of time.

In the induction mode, the auxiliary switching valve SV4 is in its on state. This causes the pressure sensor 19 to be connected to the branch passage 16, thereby allowing piping to be checked for abnormality, etc. Concentration of gas as detected by the gas sensor 18 is displayed in a monitor of the main unit 10.
In the induction mode, the exhalation valve SV3 as well as the switching valve SV2 is maintained in its off state.

When the small animal A is anesthetized, the operational mode switch is set to a maintenance mode. This closes the bypass valve SV1, thereby causing the flow rate of the anesthetic gas to be controlled at a set flow rate by the mass flow controller 12c. The anesthetic gas flows into the induction chamber 20 through the branch passage 16, the second outlet port 10c, the supply tube 60 and the mask 61. The flow rate and concentration of the anesthetic gas are lower in the maintenance mode than in the induction mode. In the maintenance mode, the switching valve SV2 and the exhalation valve SV3 are maintained in the off states.

In the induction mode and the maintenance mode described above, anesthetic agents of the anesthetic gas supplied into the induction chamber 20 are conveyed to the anesthetic gas absorber 30 via the exhaust tube 70. The anesthetic agents are absorbed by the anesthetic gas absorber 30 and not being emitted to the atmosphere in the laboratory.

The small animal A is removed from the induction chamber 20 in the maintenance mode, and is placed on the intubation table unit 40 on its back as shown in FIG. 14. Specifically, the small animal A is placed in the receiving space 48 formed with the support blocks 46, 47 as shown in FIG. 7. The mask 61 is removed from the induction chamber 20 and attached to the clamping tool 45c of the support arm 45a. The support arm 45a is bent to adjust location of the mask 61 so that the mask 61clovers the nose of the small animal A. This allows the small animal A to be maintained under anesthesia. When the small animal A is sufficiently anesthetized in the induction chamber 20, this step of administering anesthesia via the mask 61 may be omitted.

While the small animal A is maintained under anesthesia via the mask 61 as described above, the laryngoscope 80 and the intubation tube which is not shown shall be prepared. During this time, the mask 61 is supported by the support arm 45a and is not needed to be held by an operator, thereby improving efficiency of the operator's work.

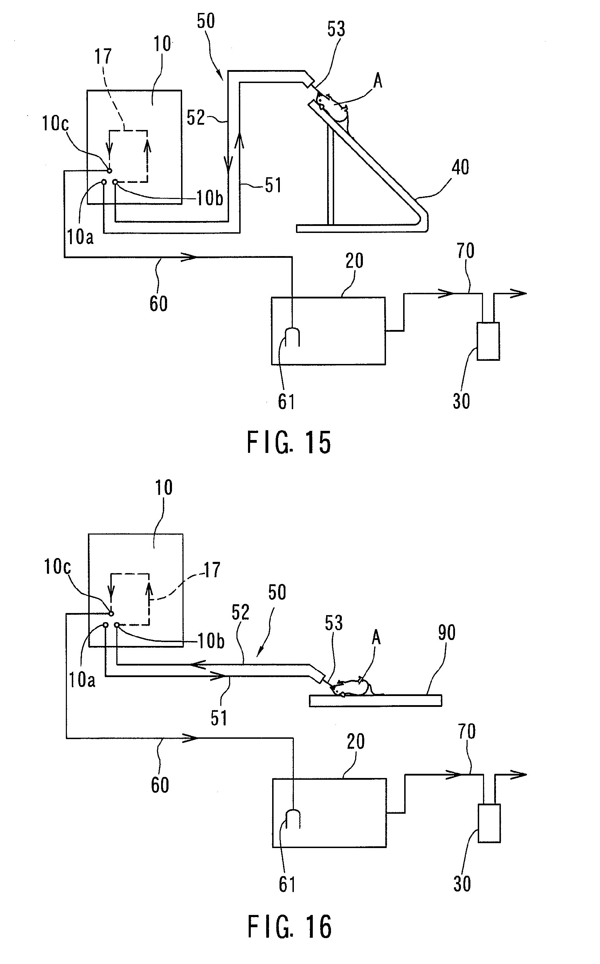

After the completion of the preparation work as described above, the mask 61 is removed from the small animal A and returned to the induction chamber 20 and the closure 22 is closed. Then, the ring 44b of the hooking tool 44 is hooked to upper front teeth of the small animal A to lift an upper jaw of the small animal A to make it easy to see into the oral cavity of the small animal A. In this condition, the intubation tube is inserted into the trachea of the small animal A using the laryngoscope 80. As shown in FIG. 15, the intubation portion 53 of the breathing circuit tube 50 is connected to the intubation tube, and the intubation work is completed.

After the completion of the intubation work, the small animal A is transferred from the intubation table unit 40 to a laboratory table 90 as shown in FIG. 16. The artificial respiration switch is turned on generally at the same time with the transferring of the small animal A. Turning on the artificial respiration switch starts an artificial respiration mode. The switching valve SV2 is in its on state in the artificial respiration mode. This causes the anesthetic gas to be supplied to a lung of the small animal A through the down stream portion 11a of the main passage 11, the first outlet port 10a, the inhalation portion 51 and the inbubation portion 53 of the breathing circuit tube 50 and the intubation tube. The auxiliary switching valve SV4 is returned to its off state. This allows the pressure in the downstream portion 11a of the main passage 11 to be detected by the pressure sensor 19.

In the artificial respiration mode, the exhalation valve SV3 repeatedly alternates between the on and off states. To be more specific, when the exhalation valve SV3 is closed, the anesthetic gas is delivered to the small animal A and the small animal A is allowed to inhale the anesthetic gas. In this condition, when the pressure detected by the pressure sensor 19 reaches a set pressure value, the exhalation valve SV3 is opened and the small animal A is allowed to exhale. The exhalation valve SV3 is closed in a set time interval, thereby artificial respiration is performed on the small animal A.

In the artificial respiration mode, the bypass valve SV1 is closed, and the flow rate of the anesthetic gas is controlled at a set flow rate by the mass flow controller 12c. The set flow rate and concentration of the anesthetic agents are lower in the artificial respiration mode than in the induction mode.

In the artificial respiration mode, gas exhaled by the small animal A, with the concentration of anesthetic agent slightly reduced, is returned to the return port 10b through the exhalation portion 52 of the breathing circuit tube 50. The gas is further conveyed through the communication passage 17 and the exhalation valve SV3 to reach the second outlet port 10c, and the gas is further conveyed from the second outlet port 10c to the induction chamber 20 through the supply tube 60 and processed by the anesthetic gas absorber 30. In this arrangement, the laboratory can be prevented from being contaminated with the anesthetic gas. The concentration of the anesthetic gas is detected by the gas sensor 18 and displayed in the monitor.
As mentioned above, an experiment or an operation is performed on the small animal A in the artificial respiration mode, with the small animal A maintained under anesthesia and artificial respiration.

The small animal A often wakes up from anesthesia during the intubation work or the experiment, causing the intubation tube to be removed from the small animal A. This makes it difficult to continue the experiment or the operation. In such a case, as shown in FIG. 13, the small animal A is returned to the induction chamber 20 with the mask 61 received in the induction chamber 20. In the same manner as in the induction mode, anesthetic gas of high concentration is supplied to the induction chamber 20 at a high flow rate to put the small animal A to sleep again and the steps illustrated in FIGS. 14 to 16 are repeated.

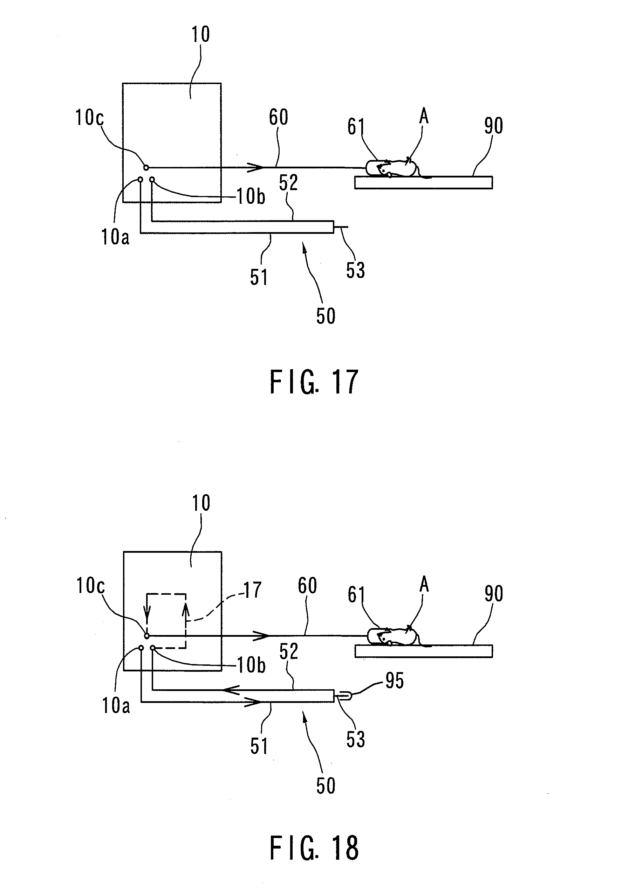

When the small animal A wakes up, as shown in FIG. 17, the mask 61 may be removed from the induction chamber 20 and placed over the nose of the small animal A to anesthetize the small animal A again. At this time, the solenoid valve SV2 is switched off such that the anesthetic gas is supplied to the mask 61 through the branch passage 16, the second outlet port 10c and the supply tube 60. At this time, it is preferable that the flow rate and the concentration of the supplied anesthetic gas are higher than in the artificial respiration mode. The anesthesia may be administered on the intubation table unit 40.

When the small animal A wakes up, the small animal A may be put to sleep with the anesthetic by removing the mask 61 from the induction chamber 20 and placing the mask 61 over the small animal A as shown in FIG. 18 without switching the solenoid valve SV2 off. In this case, the anesthetic gas is supplied to the mask 61 through the first outlet port 10a, the breathing circuit tube 50, the return port 10b, the communication passage 17, and further through the second outlet port 10c and the supply tube 60. It is preferable that the flow rate and the concentration of the anesthetic gas supplied in this way are higher than in the artificial respiration mode. It is also preferable that the intubation portion 53 is covered with a cap 95.

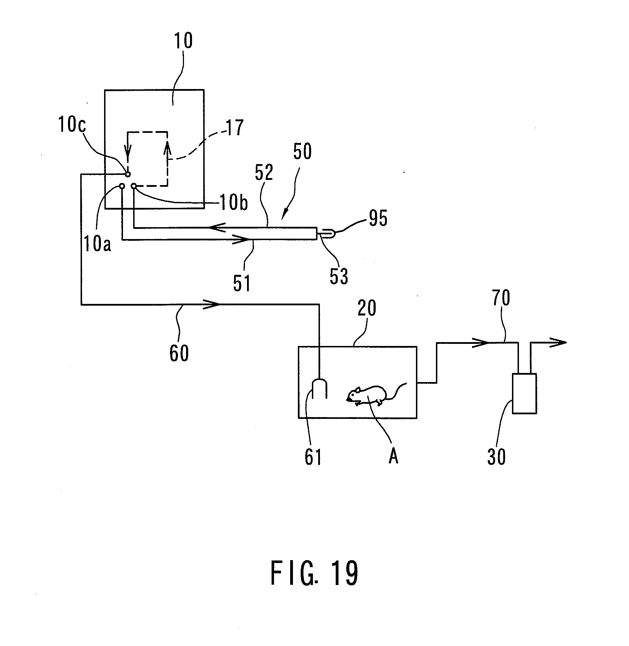

Alternatively, without switching the solenoid valve SV2 off and without removing the mask 61 from the induction chamber 20, the small animal A may also be returned to the induction chamber 20 and put to sleep with the anesthetic as shown in FIG. 19. The anesthetic gas is conveyed in the similar manner as shown in FIG. 18, and thus description thereof is omitted.

After the experiment or the operation has been performed on the small animal A, the small animal A is returned to the induction chamber 20 and an awake mode is selected by a switching operation. In the awake mode, the liquid anesthetic is not supplied, and opening of the bypass valve SV1 causes a great amount of fresh air to be supplied to the induction chamber 20 through the branch passage 16 and the second outlet port 10c, and further through the supply tube 60 and the mask 61.

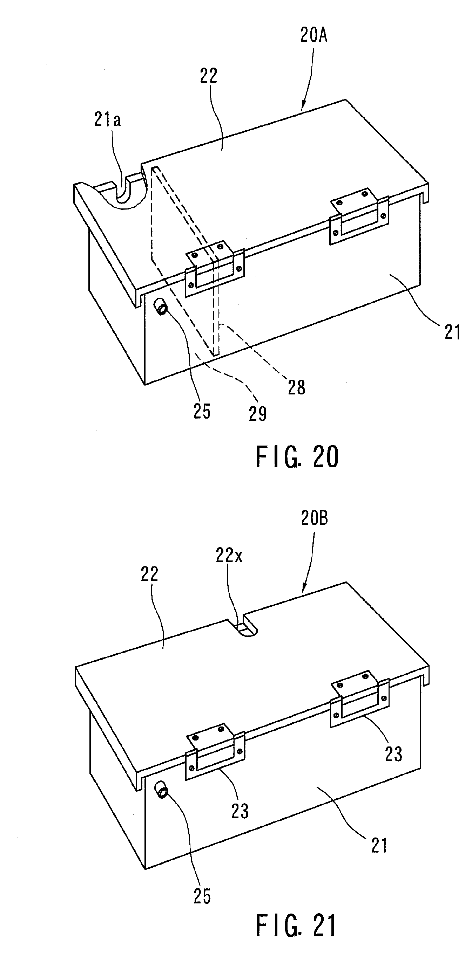

FIG. 20 illustrates another embodiment of the induction chamber 20A. An inner space of the induction chamber 20A is divided by a removable divider plate 28 to define a small space 29. In this way, a mouse can be efficiently induced to anesthesia in the small space 29. To accommodate a rat, the divider plate 28 is removed. In the example illustrated in FIG. 20, opposing inner side surfaces of the chamber main body 21 have vertical grooves (not shown) formed therein, the vertical grooves being provided for fitting opposite side edges of the divider plate 28 therein.

FIG. 21 illustrates still another induction chamber 20B. In the induction chamber 20B, a notch 22x for receiving the supply tube 60 is formed only in the edge of the closure 22.

While preferred embodiments of the present invention have been described, various modifications can be made without departing from the spirit or scope of the following claims. For example, the downstream end of the supply tube may be directly connected to the induction chamber without the mask attached to the downstream end.
Operation of the main unit for artificial respiration is not limited to the embodiment described above. For example, the anesthetic gas may be delivered to the small animal at a set flow rate and the closing and the opening of the exhalation valve may be time-controlled without depending on the pressure detected by the pressure sensor. Artificial respiration may be performed by means other than the exhalation valve.
Anesthetic gas treatment is not limited to the anesthetic gas absorption by the absorber. The anesthetic gas may be chemically changed or chemically broken down. The anesthetic gas treatment section may be a tube that discharges the anesthetic gas of the induction chamber to the outdoor air.
Oxygen may be supplied to the anesthetic gas supply section to supply oxygen-rich anesthetic gas or anesthetic gas composed of oxygen and anesthetic agents.
The induction chamber may have greater dimensions so that a plurality of small animals can be accommodated therein to be anesthetized at the same time.

### INDUSTRIAL APPLICABILITY

The present invention may be applied to a system for an experiment using a small animal, in which the small animal is given artificial respiration while being administered anesthesia.

## Claims

1. An anesthesia system comprising:
(a) a main unit (10) having a first outlet port (10a), a second outlet port (10c), a return port (10b) and a communication passage (17) connecting the return port and the second outlet port, the main unit supplying anesthetic gas through the first outlet port and providing artificial respiration;
(b) an induction chamber (20) for induction of the anesthetic gas, the induction chamber being able to receive a small animal therein;
(c) an anesthetic gas treatment section (30) connected to the induction chamber;
(d) a breathing circuit tube (50) having an inhalation portion (51), an exhalation portion (52) and an intubation portion (53) connected to a downstream end of the inhalation portion and an upstream end of the exhalation portion, the inhalation portion having an upstream end connected to the first outlet port (10a) of the main unit (10), the exhalation portion having a downstream end connected to the return port (10b) of the main unit; and
(e) a supply tube (60) having an upstream end connected to the second outlet port (10c) of the main unit (10) and having a downstream end connected to the induction chamber (20), the supply tube conveying the anesthetic gas from the second outlet port to the induction chamber.

2. An anesthesia system according to claim 1, wherein the main unit (10) comprises a first passage (11a) connected to the first outlet port (10a) and a second passage (16) connected to the second outlet port (10c), and wherein the anesthetic gas is selectively supplied either through the first passage (11a) or through the second passage (16).

3. An anesthesia system according to claim 1, wherein the main unit (10) has a main passage (11) connected to the first outlet port (10a), the main passage is provided with an anesthetic gas supply section (15) for supplying the anesthetic gas, the communication passage (17) is provided with an exhalation valve (SV3) for opening and closing the communication passage, and the artificial respiration is performed by repeatedly opening and closing the exhalation valve.

4. An anesthesia system according to claim 3, wherein the main unit (10) has a branch passage (16) branched from the main passage (11) at a branching point in a downstream side of the anesthetic gas supply section (15) and connected to the second outlet port (10c), wherein the main unit (10) further has a switching valve (SV2) provided at the branching point, and wherein the switching valve selects between a downstream portion (11a) of the main passage connected to the first outlet port (10a) and the branch passage connected to the second outlet port.

5. An anesthesia system according to claim 4, wherein the anesthetic gas supply section (15) has an air pump (12b), a flow control section (12c) and a liquid anesthetic vaporizing section (14a), wherein the air pump, the flow control section and the liquid anesthetic vaporizing section are disposed in the main passage (11) and arranged from upstream to downstream of the main passage, and wherein the anesthetic gas supply section further has a bypass passage (13) connected to the main passage bypassing the flow control section and a bypass valve (SV1) disposed in the bypass passage for opening and closing the bypass passage.

6. An anesthesia system according to any one of claims 1 to 5, wherein a mask (61) for covering a nose of a small animal is attached to a downstream end of the supply tube (60) and wherein the downstream end of the supply tube is removably connected to the induction chamber (20) through the mask.

7. An anesthesia system according to claim 6, wherein the induction chamber (20) has a chamber main body (21) having a top opening and a closure (22) for opening and closing the top opening, and wherein the downstream end of the supply tube (60) is connected to the induction chamber through the mask (61) received in the induction chamber.

8. An anesthesia system according to claim 7, wherein the chamber main body (21) has a notch (21a) formed in an upper edge of a peripheral wall thereof, the notch being provided for receiving the supply tube (60).

9. An anesthesia system according to claim 7, wherein the closure (22) of the induction chamber (20) has a notch (22x) formed in an edge thereof, the notch being provided for receiving the supply tube (60).

10. An anesthesia system according to claim 7, wherein the chamber main body (21) has a divider plate (28) removably disposed therein, and wherein a small space (29) is defined in the chamber main body with the divider plate.

11. An anesthesia system according to any one of claims 6 to 10, further comprising an intubation table unit (40) for placing a small animal thereon for insertion of an intubation tube to a trachea of the small animal, wherein one end of a bendable arm (45a) is attached to the intubation table unit, and wherein a clamping tool (45c) for removably clamping the mask (61) is disposed on the other end of the arm.
